(19) Europäisches Patentamt
European Patent Office
Office européen des brevets

(11) Veröffentlichungsnummer : **0 138 173**
**B1**

(12) # EUROPÄISCHE PATENTSCHRIFT

(45) Veröffentlichungstag der Patentschrift : **23.12.87**

(51) Int. Cl.⁴ : **C 07 D209/48**, C 08 K 5/41

(21) Anmeldenummer : **84111996.9**

(22) Anmeldetag : **06.10.84**

(54) **Neue Flammschutzmittel, ihre Herstellung und ihre Verwendung zur Flammfestausrüstung von Polycarbonaten.**

(30) Priorität : **18.10.83 DE 3337857**

(43) Veröffentlichungstag der Anmeldung : **24.04.85 Patentblatt 85/17**

(45) Bekanntmachung des Hinweises auf die Patenterteilung : **23.12.87 Patentblatt 87/52**

(84) Benannte Vertragsstaaten : **DE FR GB IT**

(56) Entgegenhaltungen :
**EP-A- 0 019 126**
**EP-A- 0 019 127**
**EP-A- 0 043 997**
**US-A- 3 753 679**
**CHEMICAL ABSTRACTS, Band 84, Nr. 4, 26. Januar 1976, Seite 52, Nr. 18389c, COLUMBUS, OHIO, (US).**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Kress, Hans-Jürgen, Dr.**
**Scheiblerstrasse 111**
**D-4150 Krefeld (DE)**
Erfinder : **Kircher, Klaus, Dr.**
**Alfred-Kubin-Strasse 3**
**D-5090 Leverkusen 1 (DE)**

**EP 0 138 173 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der vorliegenden Erfindung sind Phthalimidverbindungen der Formel (I)

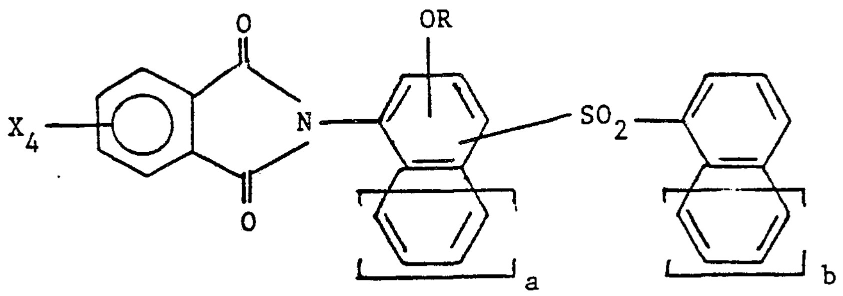

(I)

worin

X = H oder Halogen, beispielsweise Chlor oder Brom,

a und b unabhängig voneinander 0 oder 1, und

R ein $C_1$-$C_4$-Alkyl

bedeuten.

Gegenstand der vorliegenden Erfindung ist außerdem die Herstellung von Phthalimiden der Formel (I), die dadurch gekennzeichnet ist, daß ein Phthalsäureanhydrid der Formel (III)

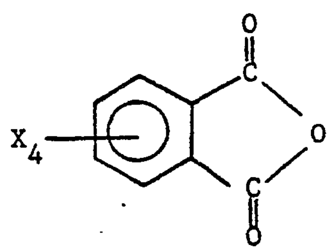

(III)

worin X die für Formel (I) genannte Bedeutung hat, mit einem Amin der Formel (II)

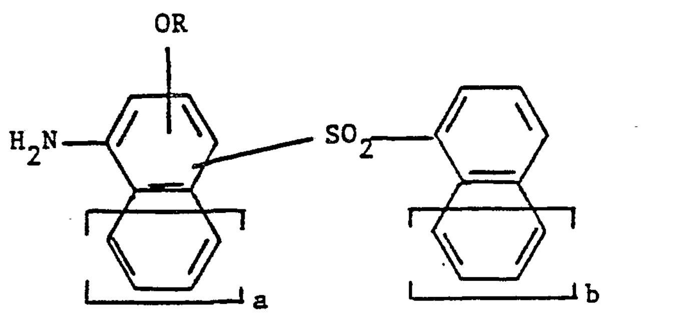

(II)

worin R, a und b die für Formel (I) angegebene Bedeutung haben, in äquimolaren Mengen bei 117 °C und unter Mitverwendung von Eisessig als Lösungsmittel und Cyclohexan als Wasserschlepper umgesetzt wird.

Die Amine der Formel (II) sind entweder literaturbekannt (siehe beispielsweise US 3 753 679) oder nach dem dort beschriebenen Verfahren erhältlich.

Die Menge an einzusetzendem Lösungsmittel beträgt pro eingesetztes Mol Amin ca. 2,5-3,0 l ; die Menge an Wasserschlepper beträgt pro Mol Amin ca. 250-300 ml.

Die neuen Phthalimidverbindungen sind in Kombination mit den bekannten Flammschutzmitteln für Polycarbonate, den Alkalisalzen organischer oder anorganischer Säuren, geeignete Synergisten zur Verbesserung der Flammwidrigkeit von thermoplastischen aromatischen Polycarbonaten, die nur aus halogenfreien phenolischen Komponenten hergestellt sind.

Gegenstand der vorliegenden Erfindung sind somit auch Flammschutzmittelkombinationen bestehend aus

a) 0,1 bis 1 Gewichtsteil eines Phthalimids der Formel (I) und

b) 0,02 bis 2 Gewichtsteilen eines Alkalisalzes einer organischen oder anorganischen Säure, insbesondere eines Natrium-, Kalium- oder Lithiumsalzes.

Als Flammschutzmittel geeignete Alkalisalze von organischen oder anorganischen Säuren sind

beispielsweise in den deutschen Offenlegungsschriften Nr. 2 703 710 Nr. 2 918 882 und Nr. 2 918 883 genannt.

Gegenstand der vorliegenden Erfindung ist außerdem die Verwendung der erfindungsgemäßen Flammschutzmittelkombination zur Flammfestausrüstung von thermoplastischen, verzweigten, aromatischen Polycarbonaten aus halogenfreien phenolischen Komponenten in Mengen von 0,1 bis 1 Gew.-%, bezogen auf thermoplastisches aromatisches Polycarbonat, an Phthalimid der Formel (I) und 0,02 bis 2 Gew.-%, bezogen auf thermoplastisches, verzweigtes, aromatisches Polycarbonat, an Alkalisalz einer organischen oder anorganischen Säure.

Gegenstand der vorliegenden Erfindung ist außerdem ein Verfahren zur Flammfestausrüstung von thermoplastischen, verzweigten, aromatischen Polycarbonaten aus halogenfreien phenolischen Komponenten, das dadurch gekennzeichnet ist, daß die Einarbeitung der Flammschutzmittel durch Mischen und anschließende Granulierung über einen Doppelwellenextruder bei einer Massetemperatur von 270-300 °C, vorzugsweise 270-280 °C erfolgt.

Die optimalen Verarbeitungsbedingungen sind derart, daß bei einer Umdrehungszahl von 40-80 Umdrehungen/min ein Durchsatz von 18 kg/h erreicht wird.

Der benutzte Doppelwellenextruder ist ein Aggregat der Firma Werner und Pfleiderer mit der Bezeichnung ZSK 53.

Gegenstand der vorliegenden Erfindung sind außerdem thermoplastische Formmassen auf Basis aromatischer, verzweigter, thermoplastischer Polycarbonate aus halogenfreien phenolischen Komponenten mit einem Gehalt an 0,1 bis 1 Gew.-% an Phthalimid der Formel (I) und an 0,02 bis 2 Gew.-% an Alkalisalz einer anorganischen oder organischen Säure, wobei sich die beiden Bereiche der Gewichtsprozente jeweils auf thermoplastisches, verzweigtes, aromatisches Polycarbonat ohne sonstige Zusätze beziehen.

Unter aromatischen, verzweigten, thermoplastischen Polycarbonaten aus halogenfreien phenolischen Komponenten ist zu verstehen, daß die für die Herstellung der Polycarbonate einzusetzenden Diphenole, Monophenole und Trisphenole, Tetraphenole oder sonstige Verzweiger keine Halogensubstituenten haben. Selbstverständlich können diese Polycarbonate, sofern sie beispielsweise nach dem Phasengrenzflächenverfahren unter Verwendung von Phosgen hergestellt sind, noch geringe Rest-ppm-Mengen an unverseiftem Chlor enthalten. Bei der nachfolgenden Charakterisierung der Polycarbonate als « halogenfrei » sollen Reste an derartig verseifbarem Halogen unberücksichtigt bleiben.

Mit Hilfe dieser neuen Flammschutzmittel sind flammgeschützte Polycarbonate mit einer UL'94 V-0-Klassifizierung möglich. Mit den erfindungsgemäßen halogenfreien Phthalimiden sind somit Cl- und Br-freie flammgeschützte Polycarbonate mit V-0 in einer Wandstärke von 1,6 mm möglich. Durch Einsatz der halogenhaltigen Verbindungen wird auch in einer Wandstärke von 0,8 mm V-0 erreicht. Zusätzlich sind die erfindungsgemäßen Phtalimide der Formel (I) durch niedrige Flüchtigkeit bei normalen Polycarbonat-Verarbeitungsbedingungen gekennzeichnet.

Die erfindungsgemäßen Polycarbonatformmassen erreichen gemäß Underwriters' Laboratories Inc., Bulletin 94, Verbrennungstests zur Klassifizierung von Materialien (nachfolgend UL 94 bezeichnet), bei Prüfkörpern mit 127 × 12,7 × 3,2 mm (1/8")- bzw. 127 × 12,7 × 1,6 mm (1/16")- bzw. 127 × 12,7 × 0,8 mm (1/32")-Maßen eine Einstufung in die Brandklasse V-0, d. h., sie sind nicht abtropfend und besitzen eine durchschnittliche Nachbrennzeit von — 5 s.

Bekannt ist, die Flammfestigkeit von Polycarbonaten durch Alkalisalzzusätze zu verbessern, wobei die Polycarbonate sowohl halogenfrei als auch halogensubstituiert sein können. (Siehe beispielsweise DE-OS 1 930 257, DE-OS 2 049 358, DE-OS 2 112 987, DE-OS 2 149 311, DE-OS 2 253 072, DE-OS 2 458 968, 2 461 063, 2 461 146 und 2 461 077).

Bekannt ist auch, die Flammfestigkeit von Polycarbonaten durch Gemische von organischen Chlorverbindungen und gewissen anorganischen Salzen zu verbessern (s. beispielsweise DE-OS 2 013 496 ; als organische Chlorverbindung wird u. a. Tetrachlorphthalsäureanhydrid als geeignet genannt).

Bekannt ist auch, mit bromierten Phthalimiden Polycarbonate flammwidrig zu machen (siehe US-Patent 3 873 587).

Bekannt ist auch der Einsatz von Phthalimidverbindungen in Kombination mit Alkalisalzzusätzen, wobei ausschließlich halogenierte Phthalimide eingesetzt werden (EP-A-0 043 997, DOS 2 707 928, DOS 2 740 850 und DOS 2 703 710).

Bekannt ist auch, organische Halogenverbindungen wie halogenierte Phthalimide in Kombination mit Alkalisalzen und mit Stoffen, die die Abtropfneigung von Polycarbonaten reduzieren, für die Flammfestausrüstung von Polymerlegierungen auf Basis von Polycarbonaten einzusetzen. (Siehe DE-OS 2 918 882 und DE-OS 2 918 883).

Bekannt ist auch, Formmassen aus verzweigten aromatischen Polycarbonaten zusammen mit Alkalisalzen und halogenierten Phthalimiden und einem zusätzlichen Bromgehalt für extreme Beflammungsbedingungen flammwidrig auszurüsten. (Siehe DE-OS 3 203 905).

Aus den genannten Literaturstellen werden jedoch unseres Erachtens die Phthalimide der Formel (I) weder vorwegegenommen, noch nahegelegt, noch deren Verwendung als Flammschutzsynergist für halogenfreie Polycarbonate.

Es sind auch bislang keine Additivkombinationen bekannt, die nach Zumischen von so geringen

Gewichtsmengen eines chlor- und bromfreien Phthalimids bereits Polycarbonatformmassen der Brandklassen V 0 gemäß UL 94 bei 1/8″ sowie 1/16″, und unter Verwendung von halogenhaltigen Produkten bei 1/32″ Wandstärke ergeben.

Die erfindungsgemäß beanspruchte Zumischung von Phthalimiden der allgemeinen Formel (I) ist deshalb besonders vorteilhaft, weil Verbindungen dieser Substanzklassen sehr thermostabil, schwerflüchtig, verseifungsstabil und in Polycarbonat gut einmischbar sind.

Geeignete Alkalisalze anorganischer Säuren im Sinne der Erfindung sind bespielsweise solche von anorganischen Protonensäuren. Anorganische Protonensäuren im Sinne der Erfindung sind Brönsted-Säuren, die Alkalisalze bilden können (zum Ausdruck « Brönsted-Säure » vgl. Fieser & Fieser « Organic Chemistry », 1965, S. 595, Interscience Publishers N.Y., USA), wie zum Beispiel meta-, ortho- oder pyro-Phosphorsäure und Protonensäuren komplexer Fluormetallverbindungen.

Geeignete Alkalisalze organischer Säuren sind im Sinne der Erfindung solche von organischen Brönsted-Säuren mit mindestens einem Kohlenstoffatom, die Alkalisalze bilden können. Solche gegebenenfalls substituierten organischen Säuren können OH- oder NH-acide Verbindungen sein, beispielsweise Sulfonsäuren, Phosphonsäuren, Thiophosphonsäuren, NH-acide Sulfonamide oder Sulfonimide. Sie müssen mindestens ein C-Atomen haben und können vorzugsweise zwischen 2 und 30 C-Atome enthalten.

Die erfindungsgemäß geeigneten Alkalisalze sollen vorzugsweise einen pH-Wert zwischen 5 und 9, insbesondere zwischen 6,5 und 7,5 haben, gemessen an 1 gew.-%igen Lösungen oder Suspensionen der Salze in Wasser bei 20 °C.

Bevorzugte Alkalisalze sind die Kalium-, Natrium- und Lithiumsalze, insbesondere die Kaliumsalze.

Bevorzugte Alkalisalze organischer Säuren sind die Natrium-, Kalium- und Lithiumsalze, insbesondere jedoch die Kaliumsalze von organischen Sulfonsäuren und Phosphonsäuren, deren organische Reste gegebenenfalls durch Halogene, wie Fluor, Chlor oder Brom, substituiert sein können. Beispielsweise seien genannt : Natrium- oder Kaliumperfluorbutansulfonat, Natrium- oder Kaliumperfluormethansulfonat, Natrium- oder Kalium-2,5-dichlorbenzolsulfonat, Natrium- oder Kalium-2,4,5-trichlorbenzolsulfonat, Natrium- oder Kalium-(4-chlorphenyl)-phosphonat, Natrium- oder Kalium-methylphosphonat, Natrium- oder Kalium-(2-phenylethyl)-phosphonat, und Lithium-phenylphosphonat.

Bevorzugte Alkalisalze anorganischer Säuren sind die Natrium-, Kalium- und Lithiumsalze, insbesondere jedoch die Kalium-Salze von Protonensäurekomplexen wie Fluormetallverbindungen sowie von meta-, ortho- oder pyro-Phosphorsäure.

Beispielsweise seien genannt : Trinatrium- oder Trikalium-hexafluoroaluminat, Dinatrium- oder Dikalium-hexafluorotitanat, Dinatrium- oder Dikalium-hexafluorosilikat, Dinatrium- oder Dikalium-hexafluorozirkonat, Natrium- oder Kalium-pyrophosphat, Natrium- oder Kalium-metaphosphat, Natrium- oder Kalium-tetrafluoroborat, Natrium- oder Kalium-hexafluorophosphat und Natrium- oder Kalium- oder Lithiumphosphat.

Besonders geeignete Salze sind : Kalium- oder Natrium-perfluorbutansulfonat, Kalium- oder Natrium-2,5-dichlorbenzolsulfonat, Kalium- oder Natrium-2,4,5-trichlorbenzolsulfonat, Kalium-hexafluoroaluminat, Kalium-pyrophosphat, Kalium-methylphosphonat, Natrium-hexafluoroaluminat und Lithiumphenylphosphonat.

Ebenfalls sind Mischungen der Salze untereinander geeignet.

Halogenfreie aromatische, verzweigte, thermoplastische Polycarbonate im Sinne der vorliegenden Erfindung sind durch Umsetzung von halogenfreien Diphenolen, insbesondere von Dihydroxydiarylalkanen, mit Phosgen oder Diestern der Kohlensäure erhältliche Polykondensate, wobei außer den unsubstituierten Dihydroxydiarylalkanen auch solche geeignet sind, deren Arylreste in o- und/oder m-Stellung zur Hydroxylgruppe Alkylgruppen tragen, und die durch den Einbau von Mengen zwischen 0,05 und 2,0 Mol-% (bezogen auf eingesetzte Diphenole) an drei- oder mehr als dreifunktionellen Verbindungen, beispielsweise solchen mit drei oder mehr als drei phenolischen Hydroxygruppen, verzweigt sind.

Polycarbonate dieser Art und deren Herstellung sind z. B. in den deutschen Offenlegungsschriften 1 570 533, 1 595 762, 2 116 974, 2 113 347, der britischen Patentschrift 1 079 821, der US-Patentschrift 3 544 514 und in der deutschen Offenlegungsschrift 2 500 092 beschrieben.

Die halogenfreien aromatischen, verzweigten, thermoplastischen Polycarbonate haben mittlere Gewichtsmittel-Molekulargewichte $\bar{M}w$ zwischen 15 000 und 100 000, vorzugsweise zwischen 20 000 und 80 000, ermittelt durch Messung der rel. Viskosität in $CH_2Cl_2$ bei 25 °C und einer Konzentration von 0,5 g/100 ml nach entsprechender Eichung.

Geeignete halogenfreie Diphenole sind z. B. Hydrochinon, Resorcin, 4,4′-Dihydroxydiphenyl, Bis-(hydroxy-phenyl)-alkane wie beispielsweise $C_1$-$C_8$-Alkylen- bzw. $C_2$-$C_8$-Alkylidenbisphenole, Bis-(hydroxyphenyl)-cycloalkane wie beispielsweise $C_5$-$C_{15}$-Cycloalkylen- bzw. $C_5$-$C_{15}$-Cycloalkylidenbisphenole, Bis-(hydroxy-phenyl)-sulfide, -ether, -ketone, -sulfoxide oder -sulfone, ferner $\alpha,\alpha'$-Bis-(hydroxyphenyl)-diisopropylbenzol sowie die entsprechenden kernalkylierten Verbindungen. Bevorzugt sind Polycarbonate auf Basis Bis-(4-hydroxyphenyl)-propan-2.2(Bisphenol A), Bis-(4-hydroxy-3,5-dimethyl-phenyl)-propan-2.2(Tetramethylbisphenol A), Bis-(4-hydroxyphenyl)-cyclohexan-1.1(Bisphenol-Z) sowie auf Basis von Dreikernbisphenolen wie $\alpha,\alpha'$-Bis-(4-hydroxyphenyl)-p-diisopropylbenzol.

Weitere für die Herstellung der Polycarbonate geeignete halogenfreie Diphenole sind in den US-Patenten 3 028 365 und 3 275 601 beschrieben.

Einige der verwendbaren Verbindungen mit drei oder mehr als drei phenolischen Hydroxygruppen sind beispielsweise Phloroglucin, 4,6-Dimethyl-2.4.6-tri-(4-hydroxyphenyl)-hepten-2, 4.6-Dimethyl-2.4.6-tri-(4-hydroxyphenyl)-heptan, 1.3.5-Tri-(4-hydroxyphenyl)-benzol, 1.1.1-Tri-(4-hydroxyphenyl)-ethan, Tri-(4-hydroxyphenyl)-phenylmethan, 2.2-Bis-[4.4-bis-(4-hydroxyphenyl)-cyclohexyl]-propan, 2.4-Bis-(4-hydroxyphenyl-isopropyl) phenol, 2.6-Bis-(2'-hydroxy-5'-methyl-benzyl)-4-methylphenol, 2-(4-Hydroxyphenyl)-2-(2.4-dihydroxyphenyl)-propan, Hexa-(4-(4-hydroxyphenyl-isopropyl)-phenyl)-ortho-terephthalsäureester, Tetra-(4-hydroxyphenyl)-methan, Tetra-(4-(4-hydroxyphenylisopropyl)-phenoxy)-methan und 1.4-Bis-((4'.4"-dihydroxytriphenyl)-methyl)-benzol. Einige der sonstigen dreifunktionellen Verbindungen sind 2.4-Dihydroxybenzoesäure, Trimesinsäure, Cyanurchlorid, 3.3-Bis-(4-hydroxyphenyl)-2-oxo-2.3-dihydroindol und 3.3-Bis-(4-hydroxy-3-methyl-phenyl)-2-oxo-2,3-dihydroindol.

Geeignete Kettenabbrecher zur Regulierung des Molekulargewichts sind beispielsweise in bekannter Weise Phenol und Alkylphenole, die in den bekannten Mengen eingesetzt werden.

Die Herstellung der aromatischen, verzweigten, thermoplastischen Polycarbonate erfolgt in bekannter Weise beispielsweise nach dem Phasengrenzflächenverfahren oder nach dem Verfahren in homogener Lösung. Auch nach dem bekannten Umesterungsverfahren können die aromatischen, thermoplastischen Polycarbonate hergestellt sein.

Besonders bevorzugte Polycarbonate im Sinne der Vorliegenden Erfindung sind verzweigte Polycarbonate auf Basis Bisphenol-A mit einem Verzweigergehalt von 0,3 bis 1,0 Mol-%, bezogen auf Mole Bisphenol-A.

Ein geeignetes Amin der Formel II ist

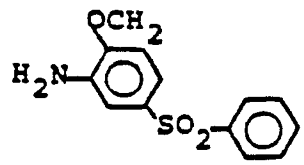

Es ist beschrieben in US-PS 3 753 679.

Geeignete Phthalimidverbindungen der Formel I sing

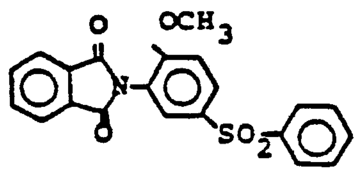

und

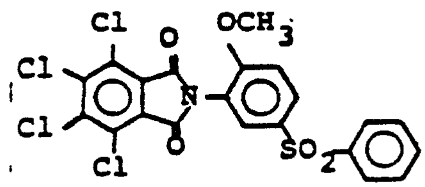

Sie sind nach dem Verfahren der Beispiele sowie nach den eingangs erwähnten allgemeinen Verfahrensbedingungen herstellbar.

Die erfindungsgemäßen Flammschutzmittelkombinationen können durch Mischen der Einzelkomponenten bzw. als Konzentrat in Polycarbonat vorab hergestellt und bis zur Verwendung gelagert werden.

Die Einarbeitung der neuen Flammschutzmittelkombination kann einzeln in Form ihrer Komponenten oder gemeinsam in die Polycarbonate erfolgen, beispielsweise durch Mischung und anschließende Granulierung des Materials über einen Doppelwellenextruder bei 270 bis 280 °C.

Die erfindungsgemäßen Formmassen auf Basis Polycarbonat und Flammschutzmittelkombination können noch andere in der Polycarbonatchemie übliche Additive enthalten, wie beispielsweise Pigmente, Farbstoffe, Füllstoffe, Stabilisatoren oder Entformungsmittel.

Die erfindungsgemäßen Formmassen können zu Formteilen oder Folien verarbeitet werden.

Die Herstellung von Formkörpern erfolgt nach dem Spritzgießverfahren bei einer Temperatur von 300-310 °C.

Die erfindungsgemäßen Formmassen können eingesetzt werden, z. B. im Elektrobereich für Schalterblenden, Steckdosen, Steckerleisten, Schaltkästen usw., im Haushaltssektor für Gehäuseteile von Bügeleisen, Kaffeemaschinen und im Großgerätebereich z. B. für Computergehäuseteile.

Beschreibung des Verbrennungstest

Nach dem UL 94-Test (Underwriter's Laboratories, Inc.) werden Polycarbonatproben zu Stäben der Abmessungen 127 × 12,7 × 3,2 (oder 1,6 oder 0,8) mm (5,00 × 0,5 × 1/8) (oder 1/16 oder 1/32) Zoll geformt. Die Stäbe werden vertikal so montiert, daß die Unterseite des Probekörpers sich 305 mm über einen Streifen Verbandsstoff befindet. Jeder Probestab wird einzeln mittels zweier aufeinanderfolgender Zündvorgänge von 10 s Dauer entzündet, die Brenneigenschaften nach jedem Zündvorgang werden beobachtet und danach die Probe bewertet. Zum Entzünden der Probe wird ein Bunsenbrenner mit einer 10 mm (3/8 inch) hohen blauen Flamme von Erdgas mit einem Wärmeinhalt von $3{,}73 \times 10^4$ $kJ/m^3$ (1.000 BTU per cubic foot) benutzt.

Die UL 94 V 0-Klassifizierung umfaßt die nachstehend beschriebenen Eigenschaften von Materialien.

die gemäß der UL 94 Vorschrift geprüft wurden. Die Polycarbonate in dieser Klasse enthalten keine Proben, die länger als 10 s nach jeder Einwirkung der Testflamme brennen ; sie zeigen keine Gesamt-Flammzeit von mehr als 50 s bei der zweimaligen Flammeinwirkung auf jeden Probensatz ; sie enthalten keine Proben, die vollständig bis hinaus zu der am oberen Ende der Probe befestigten Halteklammer abbrennen ; sie weisen keine Proben auf, die die unterhalb der Probe angeordnete Watte durch brennende Tropfen oder Teilchen entzünden ; sie enthalten auch keine Proben, die länger als 30 s nach Entfernen der Testflamme glimmen.

Andere UL 94-Klassifizierungen bezeichnen Proben, die weniger flammwidrig und selbstverlöschend sind und die flammende Tropfen oder Teilchen abgeben. Diese Klassifizierungen werden mit UL 94 V 1 und V-2 bezeichnet.

Die Polycarbonate innerhalb des Bereichs dieser Erfindung zeigen in charakteristischer Weise die für eine UL 94 V-0-Klassifizierung geforderten Eigenschaften.

## Beispiele

A. Herstellung von Phthalimiden der Formel (I)

A.1 Herstellung eines Phtalimids der allgemeinen Formel (I) unter Verwendung von Tetrachlorphthalsäureanhydrid

In einem Dreihalskolben, versehen mit Thermometer, Rührer und Wasserabscheider, werden 1 600 ml Eisessig vorgelegt und auf 55 °C erwärmt. Sodann wird eine Mischung aus 171,6 g (0,6 Mol) Tetrachlorphthalsäureanhydrid und 157,8 g (0,6 Mol) 3-Amino-4-methoxy-diphenylsulfon zugegeben und kräftig gerührt. Die Reaktionsmischung wird 2,5 h bei 118 °C unter Rückfluß gehalten und anschließend abgekühlt. Nach Zugabe von 150 ml Cyclohexan wird das Reaktionsgemisch 6 h unter Rückfluß gehalten, wobei das Reaktionswasser im Wasserabscheider abgenommen werden kann.

Der erhaltene Feststoff wird heiß abgesaugt, mit Eisessig nachgewaschen, mit heißem Aceton aufgeschlämmt und getrocknet.

| | | | |
|---|---|---|---|
| % N theor. : 2,64 | % C theor. : 47,5 | % H theor. : 2,1 | % Cl theor. : 26,7 |
| % N gef. : 2,86 | % C gef. : 47,5 | % H gef. : 2,05 | % Cl gef. : 26,5 |

A.2 Herstellung eines Phthalimids der allgemeinen Formel (I) unter Verwendung von Phthalsäureanhydrid

Die Herstellung erfolgt gemäß A 1 unter Einsatz von Phthalsäureanhydrid.

| | | |
|---|---|---|
| % N theor. : 3,56 | % C theor. : 64,1 | % H theor. : 3,82 |
| % N gef. : 3,54 | % C gef. : 64,0 | % H gef. : 3,84 |

B. Flammwidrige, verzweigte Polycarbonate

Ein verzweigtes Polycarbonat auf Basis Bisphenol A, 0,5 Mol-% 3,3-Bis-(4-hydroxy-3-methylphenyl)-2-oxo-2,3-dihydroindol, 3,0 Mol-% Phenol als Kettenabbrecher und Phosgen mit einer Losungsviskosität von 1,31 (gemessen in $CH_2Cl_2$ bei 25 °C und in einer Konzentration von 0,5 g/100 ml) wurde mit 0,1 % n-Perfluorbutansulfonsäure-K-Salz beziehungsweise mit den erfindungsgemäßen Phthalimiden vermischt, extrudiert und gemäß UL 94 in Dicken von 3,2 mm, 1,6 mm und 0,8 mm auf seine Brandwidrigkeit untersucht.

Das Ergebnis zeigt folgende Tabelle :

Beispiele

| PC (B) % | PFS % | PI % | Cl PI % | UL 94 V 3,2 mm | 1,6 mm | 0,8 mm |
|---|---|---|---|---|---|---|
| 99,9 | 0,1 | | | V-0 | V-2 | V-2 |
| 99,4 | 0,1 | 0,5 | | V-0 | V-0 | V-2 |
| 99,4 | 0,1 | | 0,5 | V-0 | V-0 | V-0 |

PC (B) : Polycarbonat gemäß Beispiel B
PFS : n-Perfluorbutansulfonsäure-K-Salz
PI : Phthalimid auf Basis 3-Amino-4-methoxy-diphenylsulfon gemäß A. 2
Cl PI : Tetrachlorphthalimid auf Basis 3-Amino-4-methoxy-diphenylsulfon gemäß A. 1

**Patentansprüche**

1. Phthalimide der Formel (I)

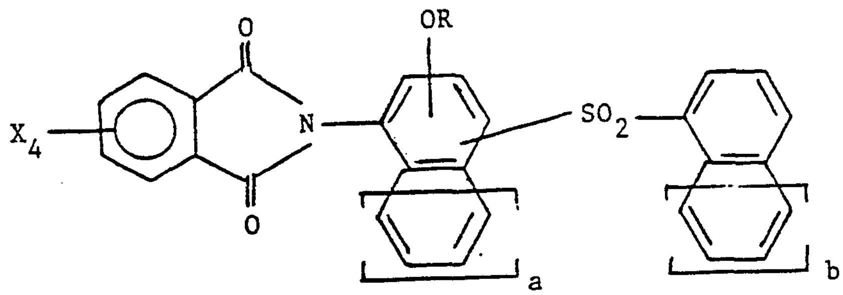

(I)

worin

X H oder Halogen,

a und b, unabhängig voneinander, 0 oder 1,

R ein $C_1$-$C_4$-Alkyl

bedeuten.

2. Verfahren zur Herstellung von Phthalimiden der Formel I, dadurch gekennzeichnet, daß ein Phthalsäureanhydrid der Formel (III)

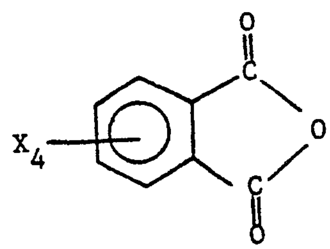

(III)

worin X die für Formel (I) genannte Bedeutung hat, mit einem Amin der Formel (II)

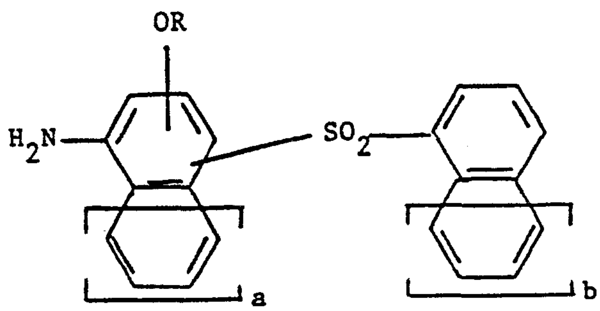

(II)

worin R, a und b die für (I) angegebene Bedeutung haben, in äquimolaren Mengen bei 117 °C und unter Mitverwendung von Eisessig als Lösungsmittel und Cyclohexan als Wasserschlepper umgesetzt wird.

3. Flammschutzmittelkombinationen bestehend aus

a) 0,1 bis 1 Gewichtsteil eines Phthalimids der Formel (I) des Anspruchs 1 und

b) 0,02 bis 2 Gewichtsteilen eines Alkalisalzes einer organischen oder anorganischen Säure.

4. Verwendung der Flammschutzmittelkombination des Anspruchs 3 zur Flammfestausrüstung von thermoplastischen, verzweigten, aromatischen Polycarbonaten aus halogenfreien, phenolischen Komponenten in Mengen von 0,1 bis 1 Gew.-%, bezogen auf Polycarbonat, an Phthalimid der Formel (I) des Anspruchs 1 und 0,02 bis 2 Gew.-%, bezogen auf Polycarbonat, an Alkalisalz einer organischen oder anorganischen Säure.

5. Verfahren zur Flammfestausrüstung von thermoplastischen, verzweigten, aromatischen Polycarbonaten aus halogenfreien, phenolischen Komponenten, dadurch gekennzeichnet, daß die Einarbeitung der Flammschutzmittel einzeln oder als Gemisch durch Mischen und anschließende Granulierung über einen Doppelwellenextruder bei einer Massetemperatur von 270-300 °C, vorzugsweise 270-280 °C erfolgt und bei einer Umdrehungszahl von 40-80 Umdrehungen/min ein Durchsatz von 18 kg/h erreicht wird.

6. Thermoplastische Formmassen auf Basis aromatischer, verzweigter, thermoplastischer Polycarbonate aus halogenfreien, phenolischen Komponenten mit einem Gehalt an 0,1 bis 1 Gew.-% Phthalimid der Formel (I) des Anspruchs 1 und an 0,02 bis 2 Gew.-% eines Alkalisalzes einer anorganischen oder

organischen Säure, wobei sich die beiden Bereiche der Gewichtsprozente jeweils auf thermoplastisches, verzweigtes, aromatisches Polycarbonat ohne sonstige Zusätze beziehen.

7. Phthalimid gemäß Anspruch 1, dadurch gekennzeichnet, daß es folgende Formel hat :

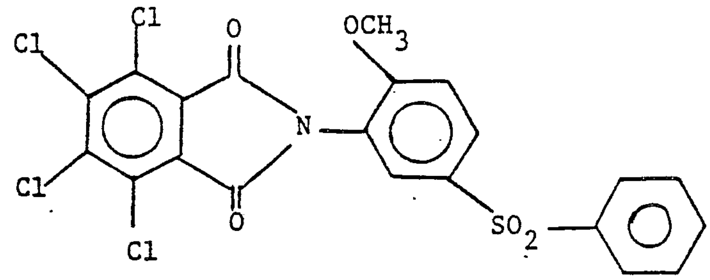

8. Phthalimid gemäß Anspruch 1, dadurch gekennzeichnet, daß es folgende Formel hat :

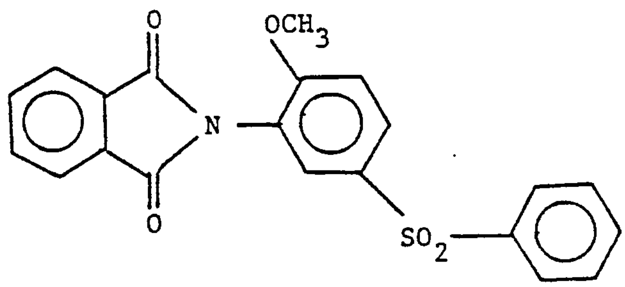

**Claims**

1. Phthalimides of the formula (I)

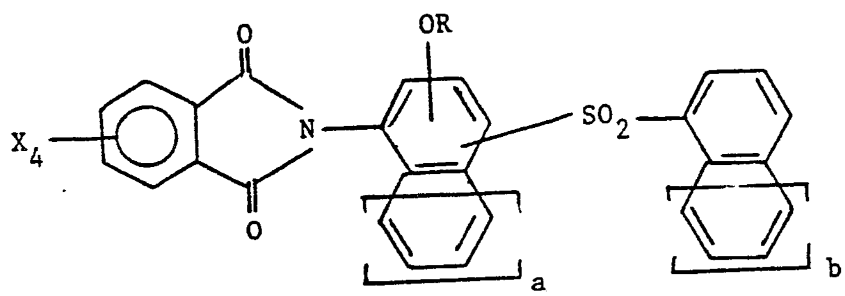

(I)

wherein

X denotes H or halogen,

a and b, independently of each other, denote 0 or 1, and

R denotes a $C_1$-$C_4$-alkyl.

2. Process for the production of phthalimides of the formula I, characterised in that a phthalic anhydride of the formula (III)

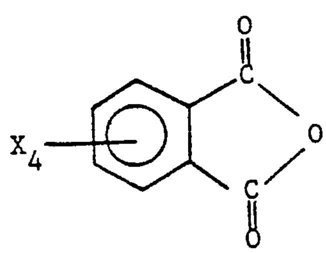

(III)

wherein X has the meaning given for formula (I), is reacted with an amine of the formula (II)

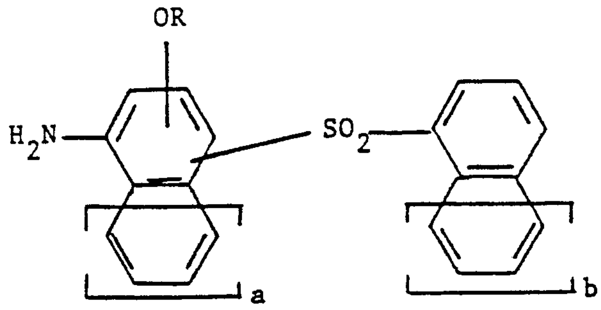

(II)

wherein R, a and b have the meaning given for (I), in equimolar quantities at 117 °C and with the concomitant use of glacial acetic acid as a solvent and cyclohexane as a water-entraining agent.

3. Flameproofing agent combinations consisting of

a) 0.1 to 1 part by weight of a phthalimide of the formula (I) of Claim 1 and

b) 0.02 to 2 parts by weight of an alkali metal salt of an organic or inorganic acid.

4. Use of the flameproofing agent combination of Claim 3 for providing thermoplastic, branched, aromatic polycarbonates obtained from halogen-free, phenolic components, with a flameproof finish in quantities of 0.1 to 1 % by weight, based on polycarbonate, of a phthalimide of the formula (I) of Claim 1 and 0.02 to 2 % by weight, based on polycarbonate, of an alkali metal salt of an organic or inorganic acid.

5. Process for providing thermoplastic, branched, aromatic polycarbonates obtained from halogen-free, phenolic components, with a flameproof finish, characterised in that the flameproofing agents are incorporated individually or in the form of a mixture by mixing and subsequent granulation via a twinscrew extruder at a stock temperature of 270-300 °C, preferably 270-280 °C and a throughput of 18 kg/h is achieved at a speed of rotation of 40-80 revolutions/min.

6. Thermoplastic moulding compositions based on aromatic, branched, thermoplastic polycarbonates obtained from halogen-free, phenolic components and having a content of 0.1 to 1 % by weight of a phthalimide of the formula (I) of Claim 1 and 0.02 to 2 % by weight of an alkali metal salt of an inorganic or organic acid, the two ranges for the percentages by weight each being based on the thermoplastic, branched, aromatic polycarbonate without any other additives.

7. Phthalimide according to Claim 1, characterised in that it has the following formula :

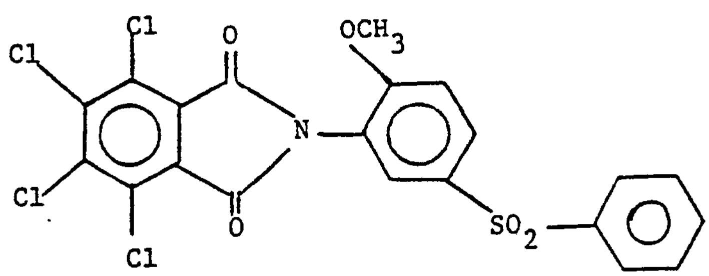

8. Phthalimide according to Claim 1, characterised in that it has the following formula :

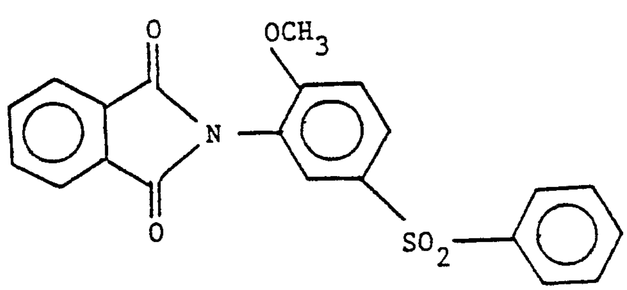

## Revendications

1. Phtalimides de formule (I)

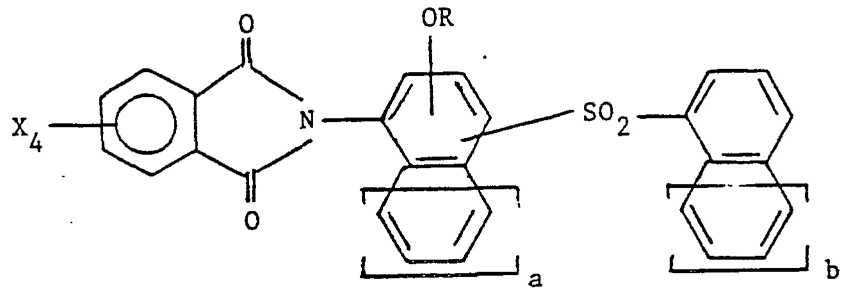

(I)

dans laquelle :

X représente H ou un halogène,

a et b représentent, indépendamment l'un de l'autre, 0 ou 1,

R représente un alkyle en $C_1$-$C_4$.

2. Procédé pour la fabrication de phtalimides de formule (I), caractérisé en ce que l'on fait réagir un anhydride phtalique de formule (III)

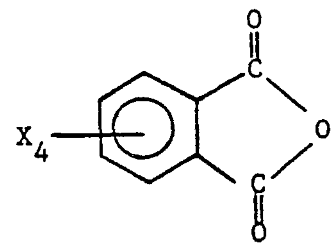

(III)

dans laquelle X a la signification indiquée pour la formule (I), avec une amine de formule (II) :

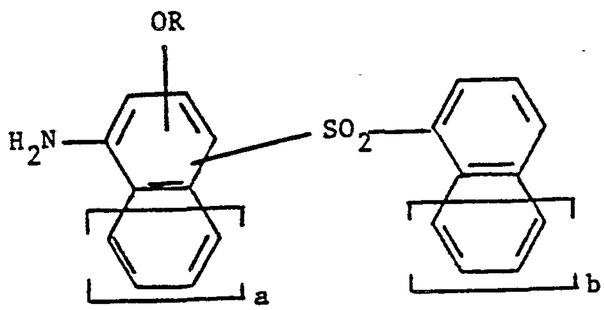

(II)

dans laquelle R, a et b ont la signification indiquée pour (I), en quantités équimolaires à 117 °C et avec utilisation simultanée d'acide acétique comme solvant et de cyclohexane comme agent d'entraînement de l'eau.

3. Combinaisons ignifugeantes consistant en :

a) 0,1 à 1 partie en poids d'un phtalimide de formule (I) selon la revendication 1, et

b) 0,02 à 2 parties en poids d'un sel alcalin d'un acide organique ou inorganique.

4. Utilisation de la combinaison ignifugeante selon la revendication 3 pour l'ignifugation de polycarbonates aromatiques ramifiés thermoplastiques dérivés de composants phénoliques non halogénés, en quantités de 0,1 à 1 % en poids, par rapport au polycarbonate, de phtalimide de formule (I) selon la revendication 1 et 0,02 à 2 % en poids, par rapport au polycarbonate, d'un sel alcalin d'un acide organique ou inorganique.

5. Procédé pour l'ignifugation de polycarbonates aromatiques ramifiés thermoplastiques dérivés de composants phénoliques non halogénés, caractérisé en ce que l'incorporation des agents ignifugeants s'effectue isolément ou en mélange par mélange suivi de granulation dans une extrudeuse à deux arbres à une température de la masse de 270-300 °C, de préférence 270-280 °C, et que l'on atteint un débit de 18 kg/h à une vitesse de rotation de 40-80 tr/min.

6. Matières à mouler thermoplastiques à base de polycarbonates aromatiques ramifiés thermoplastiques dérivés de composants phénoliques non halogénés, contenant 0,1 à 1 % en poids d'un phtalimide de formule (I) selon la revendication 1 et 0,02 à 2 % en poids d'un sel alcalin d'un acide inorganique ou organique, les deux gammes de pourcentage pondéraux étant rapportées chaque fois au polycarbonate aromatique ramifié thermoplastique, sans autres additions.

7. Phtalimide selon la revendication 1, caractérisé en ce qu'il répond à la formule suivante :

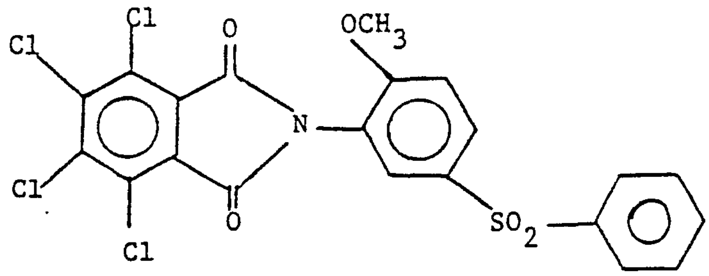

8. Phtalimide selon la revendication 1, caractérisé en ce qu'il répond à la formule suivante :